(19) Europäisches Patentamt

European Patent Office

Office européen des brevets

(11) **EP 1 104 680 A1**

(12) **EUROPEAN PATENT APPLICATION**
published in accordance with Art. 158(3) EPC

(43) Date of publication:
**06.06.2001 Bulletin 2001/23**

(51) Int Cl.⁷: **A61L 15/64**

(21) Application number: **99933226.5**

(86) International application number:
**PCT/JP99/04156**

(22) Date of filing: **03.08.1999**

(87) International publication number:
**WO 00/09175 (24.02.2000 Gazette 2000/08)**

(84) Designated Contracting States:
**AT BE CH CY DE DK ES FI FR GB GR IE IT LI LU MC NL PT SE**

(30) Priority: **14.08.1998 JP 22955198**

(71) Applicants:
- **Tapic International Co., Ltd.**
  **Tokyo 105-0001 (JP)**
- **Shimizu, Yasuhiko**
  **Uji-shi, Kyoto 611-0002 (JP)**

(72) Inventors:
- **SHIMIZU, Yasuhiko**
  **Uji-shi, Kyoto 611-0002 (JP)**
- **YAMAMOTO, Yasumichi**
  **Takamatsu-shi, Kagawa 761-0104 (JP)**
- **MATSUMOTO, Kazuya**
  **Kyoto-shi, Kyoto 606-8392 (JP)**

(74) Representative: **Stuart, Ian Alexander et al**
**MEWBURN ELLIS**
**York House**
**23 Kingsway**
**London WC2B 6HP (GB)**

(54) **TOPICALLY ABSORBENT HEMOSTATIC MATERIAL**

(57)    A topically absorbent hemostatic material which has an excellent adhesiveness to a bleeding wound face, is capable of exerting a sufficient and stable hemostatic effect, has no toxicity to a living body, is safely and quickly degraded in *vivo* after using and can be easily handled. This material is characterized in that a fiber surface made of a highly biocompatible and biodegradable material is coated with extracted collagen.

EP 1 104 680 A1

**Description**

Technical Field

The present invention relates to a local absorbent hemostatic material having biocompatibility used in the field of surgery, and comprised of a material that can be absorbed and degraded in the living body, and more particularly, to a local absorbent hemostatic material that can be rapidly and effectively adapted to capillary hemorrhage from parenchymal organs or hemorrhage from anastomosed portion of blood vessels.

Background Art

Hemostatic methods during surgical procedures include astriction, ligation, electrocoagulation as well as the application of physiologically active substances such as thrombin and fibrin paste. Ligation or electrocoagulation are typically used for arterial hemorrhage in which the source of the hemorrhage is well-defined, while ligation or astriction alone are adequate for venous hemorrhage and enable hemorrhage to be stopped easily. However, there are cases in which these hemostatic methods are not effective against capillary hemorrhage from parenchymal organs or hemorrhage from anastomosed portion of blood vessels. Hemostasis is particularly difficult in cases of being hemorrhage tendencies in surgery performed using anticoagulant such as heparin in the field of cardiovascular surgery and during hepatic insufficiency, or during surgery accompanied by a wide-ranging avulsed procedure even in the case of general surgery. In such cases, a local absorbent hemostatic material promotes the blood coagulation reaction simply by coming in contact with the hemorrhaging surface, and stops hemorrhage by rapidly forming thrombi. This local absorbent hemostatic material is effective since it reduces surgery time while also preventing recurrence of hemorrhage following surgery, thereby contributing to safe postoperative management. Examples of ideal characteristics sought after in such local absorbent hemostatic materials include: (1) high hemostatic capacity, (2) absence of toxicity to the living body, (3) absence of antigenicity, (4) able to be degraded and absorbed in the living body, (5) similarity to living body tissue, (6) able to withstand special uses, and (7) low production cost (as advocated by Martin E. Silverstein et al.).

On the other hand, local absorbent hemostatic materials available at present consist of those made of cellulose (hereinafter referred to as hemostatic material 1), those made of gelatin (hereinafter referred to as hemostatic material 2), those made of atherocollagen (hereinafter referred to as hemostatic material 3), and those made of microfibrous collagen (hereinafter referred to as hemostatic material 4). Although hemostatic material 1 is inexpensive and has the advantage of excellent handling ease, since the material itself lacks physiologically active action (action that causes the formation of thrombi by adhering and aggregating platelets and promoting blood coagulation by platelet factor III released from the aggregated platelets), its capacity to stop hemorrhage is inadequate. Hemostatic materials 2 and 3 have the problem of coming off easily due to its adhesion to a surface of hemorrhage wound being impaired as a result of gelation of its surface when it contains blood. Moreover, hemostatic materials of a type belonging to hemostatic material 3 have problems with toxicity to the living body due to chemical substances as a result of using crosslinking agents (polyepoxy compounds) for fibrinogenesis of the atherocollagen. In addition, hemostatic materials of the type belonging to hemostatic material 4 cannot be expected to demonstrate adequate hemostatic effects since the hemostatic material itself applied to wound surface is washed away and scattered with the blood because the form of fine fibers of collagen is flake-like, while also presenting handling difficulties as a result of easily adhering to the hands and pincettes during use due to being susceptible to the effects of static electricity.

Disclosure of the Invention

The object of the present invention is to provide a local absorbent hemostatic material that solves the problems of local absorbent hemostatic materials of the prior art, namely a local absorbent hemostatic material having excellent adhesion to a surface of wound, sufficient and stable hemostatic performance, is not toxic to the living body, is safely and rapidly degraded and absorbed in the living body after use and can be handled easily.

That is to say, the present invention is a local absorbent hemostatic material coated the surface of fibers composed of a material having biocompatibility and which can be degraded and absorbed in the living body, with extracted collagen.

Here, examples of the materials having the above biocompatibility that can be degraded and absorbed in the living body include polyglycolic acid, polylactic acid (including condensates with polysaccharides), copolymer of glycolic acid and lactic acid, polydioxanone, copolymer of glycolic acid and trimethylene carbonate, mixtures of polyglycolic acid and polylactic acid (including condensates with polysaccharides), and oxycellulose.

On the other hand, that produced by coating a hydrochloric acid solution of extracted collagen onto the surface of fibers composed of a material having the above biocompatibility and which is able to be degraded and absorbed in the living body followed by performing an air-drying procedure (namely amolphous collagen, which is normally adequate in this form), or further performing a freezing or freeze-drying procedure to form ultrafine fibers (effective in the case of seeking greater resistance against gelation due to contact with blood), followed by thermal dehydration crosslinking (which imparts resistance against dissolution by living body fluids), should be suitably selected for the above extracted collagen. In this connection, coating refers to coating in general, and is not limited to application by a brush and so forth.

The extracted collagen here refers to collagen solubilized with alkali or enzyme (examples of which include pepsin, trypsin, chymotrypsin, papain and pronase), and from which telopeptides, which are the groups that determine the antigenicity of the collagen, are removed simultaneous to solubilization. In this connection, since enzyme-solubilized collagen is able to more easily maintain its three-dimensional structure due to the delay in gelation and dissolution caused by the effect of pH in the living body as compared with alkali-solubilized collagen, treatment is preferably performed using the former method.

Brief Description of the Drawings

Fig. 1 is an electron micrograph showing the surface structure of the hemostatic material of the present invention.
Fig. 2 is an enlarged electron micrograph of the important portion of Fig. 1.

Best Mode for Carrying Out the Invention

Although there are no particular restrictions on the origin of the collagen used as the raw material of the present invention, in general, collagen can be used that is obtained from the skin, bone, cartilage, tendon, viscera and so forth of mammals (such as humans, cows, pigs, rabbits, sheep and mice). In addition, collagen-like protein can be used that is obtained from birds, fish and so forth. In addition, although there are numerous types of extracted collagen, type I collagen or mixed type I and type III collagen is preferable.

Hemostasis induced by the local absorbent hemostatic material of the present invention is performed by first physically deterring springing blood on the basis of physical strength demonstrated by the hemostatic material overall (here, although "overall" refers to the complex of fibers composed of a material having biocompatibility as a base material and which can be degraded and absorbed in the living body, and extracted collagen for coating its surface, the majority of physical strength comes from the former), followed by demonstration of the physiological hemostatic action of extracted collagen. Thus, although the form of the hemostatic may be, for example, absorbent cotton-like, flat sheet-like, non-woven fabric-like or woven fabric-like and so forth provided the above action is not impaired, absorbent cotton-like one is particularly preferable since it allows the obtaining of a high degree of adhesion to the bleeding wound regardless of the shape of the applied site.

In addition, with respect to the degree of coating of the former by the latter, it is not necessary that the surface of the former be 100% coated by the latter provided that the action of the latter is demonstrated to the desired degree.

The local absorbent hemostatic material of the present invention should be produced in the manner described below (the following example provides an explanation of the final product being in the form of absorbent cotton-like one).

(1) Preparation of fibers composed of a material having biocompatibility as a base material that can be degraded and absorbed in the living body (although it is convenient to use commercially available products already in the form of fibers, fibers can naturally also be prepared in-house from various types of raw materials, and a spinning method normally used in the production of fibers should be used as a specific method for accomplishing this, the thickness of the fibers preferably being 10-70 $\mu$m, and particularly preferable 15-45 $\mu$m);

(2) Preparation of hydrochloric acid solution of extracted collagen (naturally, after purification) (approximately 1 N and pH 3; consisting of a non-fibrous collagen solution in which collagen molecules are dispersed in the state of monomers to oligomers, and preferably having a concentration of 0.25-1 wt%, and particularly preferably about 0.3-0.5 wt%, as the collagen concentration);

(3) Coating of said collagen onto said base material fibers (this is should be performed by immersing bundles of said base material fibers into said collagen hydrochloric acid solution and shaking said fiber bundles therein to facilitate penetration of said collagen hydrochloric acid solution into said bundles of base material fibers, followed finally by lifting out said base material fiber bundles from said solution and air-drying);

(4) Crosslinking of said coated collagen (here, the bundles of said dried collagen-coated base material fibers should be heated under reduced pressure to perform so-called thermal dehydration crosslinking);

(5) Cutting of said base material fibers coated with said crosslinked collagen (in the sense of giving the final product a desired uniform fiber length of preferably 3-70 mm, and particularly preferably 5-50 mm); and,

(6) Formation of said cut, collagen-coated base material fibers into absorbent cotton-like one (performed according to routine methods, with the degree of formation, when expressed as the percentage of void, being preferably 70-99%, and particularly preferably 95-99%).

Moreover, in order to allow this to be used as a commercially distributed product,
(8) said absorbent cotton-like one is sterilized by exposing to, for example, ethylene oxide gas.

Furthermore, freezing, for imparting the hemostatic material with greater resistance against gelation caused by contact with blood as a final product, and its following freeze-drying procedures are performed between the above steps (3) and (4) (in this case, however, the above step (3) does not require an air-drying procedure).

Here, the conditions for the above thermal dehydration crosslinking are preferably about 105-150°C, and particularly preferably about 140°C, for preferably about 6-48 hours, and particularly preferably about 24 hours.

In addition, the conditions for the freezing and freeze-drying procedures performed as additional procedures are freezing at preferably about -10 to -196°C, and particularly preferably about -20°C, for preferably about 6-48 hours, and particularly preferably about 24 hours, and freeze-drying under a vacuum at preferably -40 to -80°C, and particularly preferably about -80°C, for preferably about 24-48 hours, and particularly preferably about 48 hours.

The following provides a detailed explanation of the present invention using one embodiment of the local absorbent hemostatic material of the present invention.

A absorbent cotton-like hemostatic material was prepared according to the following procedure respectively using oxycellulose for the material having biocompatibility as a base material that can be degraded and absorbed in the living body, and fine fibrous collagen for the extracted collagen that coats said base material.

(1) Preparation of Base Material
Absorbent cotton-like OXYCELL, a commercially available hemostatic material (trade name; sold by Japan Becton-Dickson) was broken up manually to prepare dispersed oxycellulose fibers.

(2) Preparation of Collagen Hydrochloric Acid Solution
Atherocollagen extracted from bovine skin by an enzymatic method (type I: 70%, type III: 30%) was dissolved in 1 N hydrochloric acid to prepare a hydrochloric acid solution of 0.5% atherocollagen.

(3) Collagen Coating of Base Material
100 ml of said collagen hydrochloric acid solution and 0.1 g of said oxycellulose fibers were placed in a homogenizer and mixed for 5 minutes at 1,000 rpm.

(4) Post-treatment 1
Said mixture was placed in a paper cup, frozen for 12 hours at -80°C and then freeze-dried for 48 hours under reduced pressure ($6.7 \times 10^{-2}$ Pa).

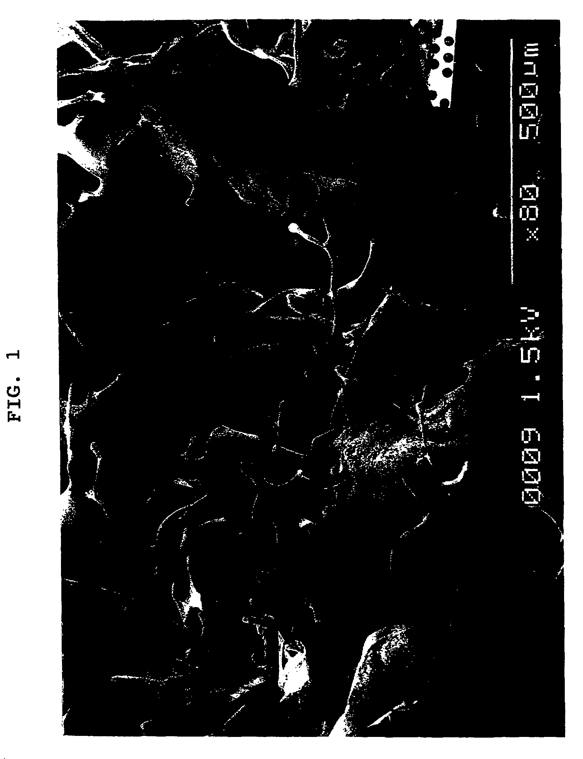

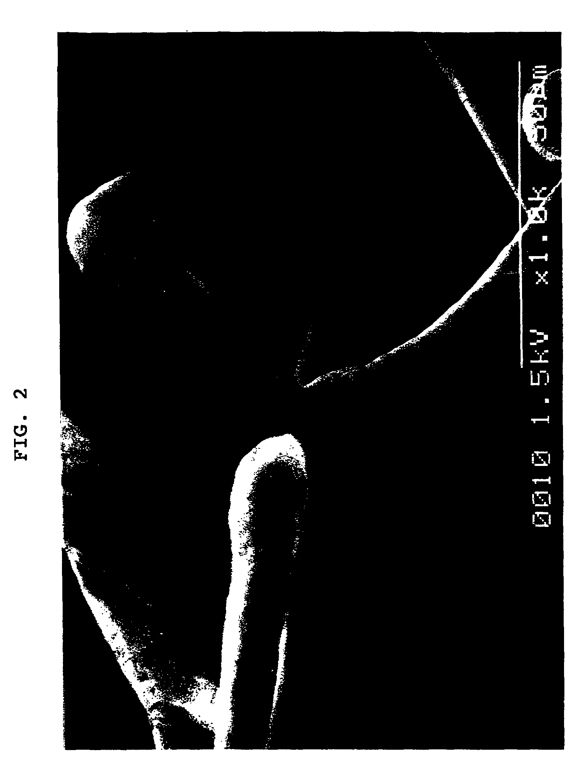

(5) Post-treatment 2
Said freeze-dried product was heated for 24 hours at 140°C under reduced pressure ($1.0 \times 10^{1}$ Pa). The resulting product was soft and sponge-like, was able to be easily disentangled or torn apart by hand into absorbent cotton-like one, and was easily applied to a surface of hemorrhage wound. (The coated state of the base material fibers by collagen is shown in Figs. 1 and 2. From these drawings, the surfaces of single oxycellulose fibers as well as multiple fibers, including between said fibers, can be clearly seen to be coated with collagen fine fibers.)

Test Example

Groups of five female beagle dogs were prepared for use as the specimens. After applying celiotomy to the specimens to expose the splen and intravenous administration of 100 u/kg of heparin, approximately 1 m$^2$ x 5 mm x 3 locations of the capsula lienis was severed followed by application of each type of hemostatic material (consisting of the above preparation for the product of the present invention, and commercially available hemostatic material 1 (absorbent cotton-like one) and commercially available hemostatic material 3 (absorbent cotton-like one)) and investigation of hemostatic performance.

Furthermore, for those specimens having a bleeding degree (BD) immediately prior to application of hemostatic material, namely $BD_0$, of less than 50, a deeper incision was made into the wound to induce bleeding so that $BD_0$ was 50 or more.

Here, $BD_0$ refers to the mean value of the long axis and short axis of a circle created on a piece of surgical cut gauze (Japanese Pharmacopoeia Gauze Type "Dekuze" (trade name) folded four times and measuring 30 x 30 cm (hereinafter referred to simply as gauze)) placed against the surface of wound immediately after formation of said surface of wound by blood absorbed for 30 seconds. Furthermore, hemostatic performance was evaluated according to the following formula using the mean value of the long axis and short axis of a circle created by placing gauze for 30 seconds on top of a hemostatic material placed against said surface of wound immediately after measurement of $BD_0$, and absorbing blood into that gauze (said mean value is referred to as $BD_t$, with t representing the elapsed time from immediately after placing the hemostatic material against said surface of wound and pressing by hand for 30 seconds, and measurements being performed at 1 minute intervals).

$$\text{Hemostasis rate after t minutes (\%)} = (1-BD_t/BD_0) \times 100$$

Hemostasis was considered to have stopped completely at the point there was only a trace of blood adhered to said gauze.

Those results are shown in Table 1.

Furthermore, although an adequate amount of hemostatic material was used at the start of hemostasis, in the case the hemostatic material came off during the course of measurement of $BD_t$, a fresh hemostatic material was added ("Amount of hemostatic material used" in the table includes hemostatic material added in this manner.) All tests were completed within 60 minutes, and additional heparin was not administered. In addition, in order to avoid effects of blood loss on measurement of hemostatic performance, the applying order of the hemostatic materials was changed in order. A hemostatic material was applied to a wound site at which hemorrhage was not stopped, and the next wound site was created and testing was continued only after confirming that hemorrhage had stopped completely. The presence or absence of recurrence of hemorrhage was confirmed by moving the splen in all cases in which hemorrhage had stopped completely at the hemorrhage wound.

The meaning of each evaluated parameter in the table is as described below.

(1) 100% Hemostasis Rate

This refers to the percentage of cases in which hemorrhage stopped completely within 10 minutes (units consist of the number of cases in which hemorrhage stopped completely for the numerator, the total number of cases for the denominator, and the percentage in parentheses). In this connection, the value of 3 for the numerator of commercially available product (1) refers to the number of cases in which hemorrhage stopped by applying an additional hemostatic material due to blood having escaped from a gap in the hemostatic material, while hemorrhage was unable to be completely stopped in the remaining two cases even if such action was taken. In addition, although hemorrhage appeared to have been stopped completely in all the cases of commercially available product (2), these results were actually obtained by being unavoidably forced to redo hemostasis (repeat hemostasis) since the hemostatic material had been lifted from the wound site. On the other hand, hemostasis was not made by not only applying an additional hemostatic material but also redoing hemostasis for the product of the present invention.

(2) Hemostasis Completion Time

This refers to the value of t for $BD_t$ at which hemorrhage was confirmed to have stopped completely (units: minutes; values in parentheses: mean values).

(3) Hemorrhage Recurrence Rate

Refer to the above explanation (units: number of cases in which hemorrhage recurred for the numerator and total number of cases for the denominator; figures in parentheses: percent).

(4) Amount of Hemostatic Material Used

Refer to the above explanation (units: grams; figures in parentheses: mean values).

(5) Separation of Hemostatic Material

This refers to the proportion of cases in which the hemostatic material separated from the wound site (units: number of cases in which separation occurred for the numerator, total number of cases for the denominator).

Table 1

|  | Product of present invention | Commercially available product (1) | Commercially available product (2) |
|---|---|---|---|
| 100% hemostasis rate | 5/5 (100) | 3/5 (60) | 5/5 (100) |
| Hemostasis completion time | 1-5 (2.8) | 3-7 (5.0) | 1-7 (4.0) |
| Hemorrhage rate recurrence rate | 0/5 (0) | 1/3 (33) | 1/5 (20) |
| Amount of hemostatic material used | 0.2-0.6(0.4) | 0.5-0.8 (0.7) | 0.4-0.7 (0.5) |
| Separation of hemostatic material | 0/5 | 0/5 | 5/5 |

Industrial Applicability

As shown in the table, the hemostatic material of the present invention demonstrated extremely superior performance as compared with the conventional products for all evaluated parameters. The hemostatic material of the present invention is particularly superior as a hemostatic material with respect to only requiring that a small amount be used, and with respect to the absence of separation of the hemostatic material.

Since the raw material of the hemostatic material of the present invention is already used as a medical material, and is produced without the use of chemicals, there is no risk of the occurrence of toxicity to the living body. Moreover, in addition to both the base material and coating material having biocompatibility, they are both safely and rapidly degraded and absorbed in the living body.

Thus, according to the present invention, a local absorbent hemostatic material can be provided that, in addition to having superior adhesion to a surface of hemorrhage wound along with adequate and stable hemostatic performance, it is free of toxicity to the living body, is safely and rapidly degraded and absorbed in the living body after use, and is easy to handle.

**Claims**

1. A local absorbent hemostatic material coated the surface of fibers composed of a material having biocompatibility and which can be degraded and absorbed in the living body, with extracted collagen.

2. The hemostatic material according to claim 1, wherein said material having biocompatibility and which can be degraded and absorbed in the living body is selected from the group consisting of polyglycolic acid, polylactic acid, copolymer of glycolic acid and lactic acid, polydioxanone, copolymer of glycolic acid and trimethylene carbonate, mixtures of polyglycolic acid and polylactic acid, and oxycellulose.

3. The hemostatic material according to claims 1 or 2, wherein said extracted collagen is the one obtained by performing an air-drying, or a freezing and freeze-drying procedures to form ultrafine fibers, followed by thermal dehydration crosslinking after coating said extracted collagen onto the surface of fibers composed of a material having the above biocompatibility and which is able to be degraded and absorbed in the living body.

FIG. 1

FIG. 2

## INTERNATIONAL SEARCH REPORT

| | International application No. |
|---|---|
| | PCT/JP99/04156 |

**A. CLASSIFICATION OF SUBJECT MATTER**
Int.Cl$^6$ A61L15/64

According to International Patent Classification (IPC) or to both national classification and IPC

**B. FIELDS SEARCHED**

Minimum documentation searched (classification system followed by classification symbols)
Int.Cl$^6$ A61L15/00-15/64

Documentation searched other than minimum documentation to the extent that such documents are included in the fields searched

Electronic data base consulted during the international search (name of data base and, where practicable, search terms used)
WPI/L

**C. DOCUMENTS CONSIDERED TO BE RELEVANT**

| Category* | Citation of document, with indication, where appropriate, of the relevant passages | Relevant to claim No. |
|---|---|---|
| X, Y | JP, 6-296673, A (Mitsubishi Rayon Co., Ltd.), 25 October, 1994 (25. 10. 94), Claims ; Par. Nos. [0009], [0013], [0016] (Family: none) | 1-3 |
| Y | JP, 6-197946, A (Yoshihiko Shimizu, Nippon Meat Pachkers,Inc., Mitsubishi Rayon Co., Ltd.), 19 July, 1994 (19. 07. 94), Claims ; Par. Nos. [0011] to [0013] ; Examples ; Comparative Examples & WO, 9409831, A1 & EP, 667167, A1 & US, 5679372, A | 1-3 |
| A | JP, 4-61862, A (Koken Co., Ltd.), 27 February, 1992 (27. 02. 92), Claims & EP, 463887, A2 | 1-3 |

☐ Further documents are listed in the continuation of Box C.    ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | |
| "O" | document referring to an oral disclosure, use, exhibition or other means | "Y" | document of particular relevance; the claimed invention cannot be considered to involve an inventive step when the document is combined with one or more other such documents, such combination being obvious to a person skilled in the art |
| "P" | document published prior to the international filing date but later than the priority date claimed | "&" | document member of the same patent family |

| Date of the actual completion of the international search | Date of mailing of the international search report |
|---|---|
| 17 September, 1999 (17. 09. 99) | 28 September, 1999 (28. 09. 99) |

| Name and mailing address of the ISA/ | Authorized officer |
|---|---|
| Japanese Patent Office | |
| Facsimile No. | Telephone No. |

Form PCT/ISA/210 (second sheet) (July 1992)